# EUROPÄISCHE PATENTANMELDUNG

(11) **EP 1 044 984 A2**
(43) Veröffentlichungstag der Anmeldung: **18.10.2000**
(21) Anmeldenummer: 00108179.3
(22) Anmeldetag: 13.04.2000
(51) Int. Cl.: C07H 1/08

(54) **RNA-Isolierungs-Kit**

(30) Priorität: 13.04.1999 DE 19916534
(71) Anmelder: GSF-Forschungszentrum für Umwelt und Gesundheit, GmbH, 85764 Oberschleissheim (DE)
(72) Erfinder: Kiefer, Evelyn, 85560 Ebersberg (DE); Heller, Werner, 85386 Eching (DE); Ernst, Dietrich, 82131 Gauting (DE); Sandermann, Heinrich, 81377 München (DE)
(74) Vertreter: Maiwald, Walter, Dr. Dipl.-Chem.

(57) **Zusammenfassung**

Die vorliegende Erfindung betrifft einen RNA-Isolierungs-Kit umfassend einen Extraktionspuffer und wenigstens ein Adsorptionsmittel, wobei der Extraktionspuffer mindestens eine im pH-Bereich von 7-9 wirksame Puffersubstanz, mindestens ein Tensid und gegebenenfalls Polyvinylpyrrolidon (PVP) oder Polyvinylpolypyrrolidon (PVPP) und weitere übliche Inhaltsstoffe wie Salze, Komplexbildner, Proteinasen, Mercaptoethanol, Polyamine und dergleichen enthält und das Adsorptionsmittel in der Lage ist, Polysaccharide zu binden. Weiterhin betrifft die vorliegende Erfindung einen Extraktionspuffer und dessen Verwendung zur Isolierung von RNA, umfassend mindestens eine im pH-Bereich von 7-9 wirksame Puffersubstanz, mindestens ein Tensid und gegebenenfalls Polyvinylpyrrolidon (PVP) oder Polyvinylpolypyrrolidon (PVPP) und weitere übliche Inhaltsstoffe wie Salze, Komplexbildner, Proteinasen, Mercaptoethanol, Polyamine und dergleichen; sowie ein Verfahren zur Isolierung von RNA aus biologischem Material, vorzugsweise aus Pflanzen.

## Beschreibung

Die vorliegende Erfindung betrifft einen Ribonukleinsäure-Isolierungs-Kit, einen Extraktionspuffer für einen RNA-Isolierungs-Kit, ein Verfahren zur Isolierung von RNA aus biologischem, vorzugsweise pflanzlichem Material sowie die Verwendung des Extraktionspuffers zur Isolierung von RNA aus biologischem Material.

Die Isolierung von Nukleinsäuren wie DNA und RNA aus pflanzlichen, tierischen oder menschlichen Zellen und Zellkulturen erfolgt in der Regel nach einem einheitlichen Grundmuster: In einem ersten Schritt werden die Zellen lysiert, wodurch die Inhaltsstoffe in Lösung gebracht werden. In den nachfolgenden Schritten werden dann einzelne Bestandteile mit verschiedensten Methoden sukzessive abgetrennt, bis schließlich nur noch der gewünschte Nukleinsäureanteil in Lösung ist.

Die Abtrennung des unweigerlich in jedem Zellenlysat vorkommenden Proteinanteils ist dabei ein besonders wichtiger Schrift. Sie kann beispielsweise durch Schütteln des Protein/Nukleinsäure-Gemisches mit Phenol und/oder Chloroform/Isoamylalkohol-Mischungen erfolgen, was zur Abtrennung des Proteins führt, oder durch Zusatz denaturierender Salze wie Guanidiniumchlorid, Guanidiniumisothiocyanat und dergleichen zum Zweck der Ausfällung des Proteinanteils aus der wäßrigen Phase. Auch durch Zusatz von Proteasen kann der Proteinanteil abgebaut und anschließend entfernt werden. Anschließend kann durch Zusatz wahlweise von DNase oder RNase die jeweils gewünschte Nukleinsäurefraktion erhalten werden. Die Trennung der Nukleinsäuren kann auch durch Ultrazentrifugation erfolgen. Zum Schutz der Nukleinsäuren vor enzymatischem Abbau während der Isolierungsprozedur muß unter sterilen, Nuclease-freien Bedingungen gearbeitet werden.

Zur Durchführung derartiger Nukleinsäureextraktionen sind im Stand der Technik eine Vielzahl von kommerziellen Extraktions-Kits bekannt, welche die für die jeweilige Anwendung notwendigen Reagentien in standardisierter Form und Menge enthalten. Die meisten im Stand der Technik bekannten Kits basieren auf einem der folgenden zwei Abtrennungsprinzipien:

Die klassischen Verfahren beruhen auf einem einstufigen Prozeß, in dem nach Zusatz eines meist Guanidiniumsalze enthaltenden Puffers eine Extraktion der abzutrennenden Begleitstoffe in eine organische Phase, meistens mit Chloroform und Phenol, erfolgt. Die in der wässrigen Phase verbleibende Nukleinsäure kann dann durch Phasenseparation abgetrennt und isoliert werden. Kommerziell erhältliche Kits nach diesem Verfahren sind beispielsweise RNAprep® (Oncor Appligen), RNAble® und RNAxel® (Eurobio), und Trizol® (Life Technologies).

Ein Nachteil dieser Verfahren, neben der Verwendung giftiger und gesundheitsschädlicher Stoffe (Guanidinisothiocyanat, Phenol, CHCl₃), ist vor allem, daß wasserlösliche Stoffe als Verunreinigung in der wäßrigen Nukleinsäure-Lösung verbleiben, welche nachfolgend in weiteren, sehr zeitaufwendigen Reinigungsschritten abgetrennt werden müssen. Dieses Problem macht die Verwendung dieser Verfahren zur Isolierung von Nukleinsäuren aus Pflanzen nahezu unmöglich, da diese meist große Mengen an Polysacchariden und ähnlichen wasserlöslichen Stoffen enthalten.

In letzter Zeit hat sich ein alternativer Prozeß immer mehr durchgesetzt, welcher auf der selektiven Adsorption von Nukleinsäuren an Siliciumdioxid-Säulen oder -Filtern basiert. Hierbei werden in einem mehrstufigen Verfahren dem Zellenlysat nacheinander verschiedene Pufferlösungen (Lyse-, Binde-, Wasch- und Elutionspuffer) zugesetzt und im letzten Schritt die gereinigte Nukleinsäure vom Filter abgetrennt. Beipiele für kommerzielle Kits nach diesem Prinzip sind NucleoSpinRNA® (Macherey-Nagel), RNAqueous® (Ambion), HighPure RNA Isolation Kit® (Boehringer) und RNeasy® (Qiagen).

Nachteilig bei diesem Isolierungsprinzip ist die Vielzahl der verwendeten Puffer (fünf und mehr) und Reinigungsschritte, was zu einem erhöhten Zeitaufwand führt. Zudem werden auch in diesen Kits meist gesundheitsschädliche Guanidiniumsalze enthaltende Puffer verwendet. Weiterhin müssen zugunsten der Reinheit der isolierten Nukleinsäuren Ausbeuteverluste hingenommen werden. Gerade im Fall von pflanzlichen Proben, die einen hohen Anteil an Stoffen mit ähnlichem Löslichkeitsverhalten wie Nukleinsäuren aufweisen, treten oftmals hohe Elutionsverluste an Nukleinsäure auf.

Für Anwendungen im Bereich der Nukleinsäureisolierung aus Pflanzen eignen sich die im Stand der Technik bekannten Kits wenn überhaupt, dann nur für Zellkulturen und gängige "Hauspflanzen" wie z.B. Tabak, Tomate, Arabidopsis, Gerste, welche nur wenige Sekundärstoffe aufweisen. Bei Pflanzen mit hohem Anteil an störenden Sekundärstoffen sind diese Methoden aufgrund der geringen erzielbaren Ausbeuten nicht einsetzbar. Zudem wird mit den herkömmlichen Methoden oftmals eine nicht funktionale RNA erhalten.

Es besteht daher ein Bedürfnis nach einem RNA-Isolierungs-Kit, welcher in einfacher Weise die quantitative Isolierung von RNA aus biologischem Material, insbesondere aus stark mit Sekundärstoffen angereichertem Material in hoher Reinheit ermöglicht.

Weiterhin besteht ein Bedürfnis nach einem einfachen, wenige Schritte umfassenden und schnell durchzuführenden Verfahren zur Isolierung von RNA in hoher Reinheit, welches die erwähnten Nachteile des Standes der Technik überwindet.

Aufgabe der vorliegenden Erfindung ist es demnach, einen RNA-Isolierungs-Kit, einen Extraktionspuffer und ein Verfahren zur Isolierung von RNA aus biologischem Material, vorzugsweise aus Pflanzen, unter Verwendung des Extraktionspuffers bzw. des Kits zur Verfügung zu stellen, welche die genannten Nachteile des Standes der Technik überwinden.

Zur Lösung dieser Aufgaben dienen die Merkmale der unabhängigen Schutzansprüche.

Vorteilhafte Ausgestaltungen sind in den jeweiligen Unteransprüchen definiert.

Die Aufgabe wird gelöst durch einen erfindungsgemäßen RNA-Isolierungs-Kit, welcher einen Extraktionspuffer und wenigstens ein Adsorptionsmittel umfaßt, wobei der Extraktionspuffer mindestens eine im pH-Bereich von 7-9 wirksame Puffersubstanz, mindestens ein Tensid und gegebenenfalls Polyvinylpyrrolidon (PVP) oder Polyvinylpolypyrrolidon (PVPP) und weitere übliche Inhaltsstoffe wie Salze, Komplexbildner, Proteinasen, Mercaptoethanol, Polyamine und dergleichen enthält und das Adsorptionsmittel in der Lage ist, Polysaccharide zu binden.

Eine weitere Lösung der Aufgabe der Erfindung besteht in einem erfindungsgemäßen Extraktionspuffer zur Isolierung von RNA, der mindestens eine im pH-Bereich von 7-9 wirksame Puffersubstanz, mindestens ein Tensid und gegebenenfalls Polyvinylpyrrolidon (PVP) oder Polyvinylpolypyrrolidon (PVPP) und weitere übliche Inhaltsstoffe wie Salze, Komplexbildner, Proteinasen, Mercaptoethanol, Polyamine und dergleichen umfaßt.

Weiterhin wird die verfahrensbezogene Aufgabe der Erfindung dadurch gelöst, daß ein Verfahren zur RNA-Isolierung aus biologischem, vorzugsweise pflanzlichem und pilzlichem Material unter Verwendung des erfindungsgemäßen RNA-Isolierungs-Kits und/oder des erfindungsgemäßen Extraktionspuffers aufgezeigt wird, welches folgende Schritte umfaßt:
- Mischen des biologischen Materials mit Extraktionspuffer,
- Inkubieren der Mischung bei erhöhter Temperatur,
- Zugabe von Lösungsmittel bzw. -gemisch, vorzugsweise von Chloroform/Isoamylalkohol, und Adsorptionsmittel aus dem RNA-Isolierungs-Kit,
- Abtrennen der wäßrigen Phase und
- Isolierung der darin enthaltenen gereinigten RNA nach üblichen Methoden.

Der erfindungsgemäße RNA-Isolierungs-Kit besteht demnach im wesentlichen aus mindestens einem Adsorptionsmittel und einem darauf abgestimmten Extraktionspuffer.

Überraschenderweise konnte gefunden werden, daß nicht die Adsorption der RNA an einem geeigneten Adsorptionsmittel, sondern die Adsorption derjenigen Sekündärstoffe am Adsorptionsmittel, welche ähnliche Lösungseigenschaften wie die Nukleinsäuren aufweisen, wesentliche Vorteile in der Isolierung von RNA aus begleitstoffreichen Gewebeproben bringt.

Den Adsorptionsmitteln kommt daher im Gegensatz zu den aus Extraktions-Kits des Standes der Technik bekannten Adsorptionsmitteln im wesentlichen die Aufgabe zu, störende Sekundärstoffe dauerhaft zu adsorbieren. Derartige Sekundärstoffe sind im Falle von Pflanzen meist Polysaccharide, phenolische Substanzen, Latex und diverse schleimartige, meist unbekannte Substanzen.

Im Zusammenhang mit der Erfindung geeignete Adsorptionsmittel umfassen unfunktionalisierte und funktionalisierte Siliciumdioxide, Borsilikate, Aluminiumoxide, Zeolithe, Tonmineralien, nichtionische und ionische organische Polymere sowie funktionalisierte oder nicht funktionalisierte Harze auf der Basis von Siliciumdioxiden, Borsilikaten, Aluminiumoxiden, organischen Polymeren und dergleichen. Bevorzugte funktionelle Gruppen der vorstehend genannten Adsorptionsmittel sind veresterbare funktionelle Gruppen wie Borsäure, Sulfonsäure, Carbonsäure, Anhydride, und dergleichen. Idealerweise werden Polysaccharide und ähnliche Begleitstoffe an dem Adsorptionsmittel kovalent gebunden.

Besonders bevorzugt als Adsorptionsmittel sind erfindungsgemäß Harze, die freie Borsäuregruppen (-B(OH)₂-Gruppen) enthalten. Derartige Harze reagieren mit Polysacchariden, indem die Borsäuregruppen der Harze mit den vicinalen Hydroxygruppen der 1,2-Dihydroxyverbindungen darstellenden Polysaccharide einen cyclischen Borsäureester bilden. Durch diesen Mechanismus werden die Polysaccharide an die Harzpartikel gebunden und aus der Probe entfernt.

Kommerziell erhältliche Adsorptionsmittel, welche im Sinne dieser Erfindung vorteilhaft verwendet werden können, sind beispielsweise das Nucleon® PhytoPure® Harz der Firma Nucleon Biosciences (vertrieben von Amersham International) zur Entfernung von Polysacchariden und Amberlite® XAD-2 (vertrieben von Serva, Sigma) zur Entfernung von Latexanteilen in stark gummihaltigen Pflanzen (z.B. Ficus Benjamina).

Das Nucleon® PhytoPure® Harz von Nucleon Biosciences enthält freie Borsäuregruppen auf einer Siliciumdioxidmatrix. Dieses Harz bildet während der Extraktion eine halbfeste Schicht zwischen einer unteren organischen Phase und einer oberen wäßrigen Phase, die die RNA enthält. Dadurch wird eine Gewinnung von hochreiner RNA mit hoher Ausbeute ohne Vielzahl von Reinigungsschritten sichergestellt.

Extraktionspuffer gemäß der vorliegenden Erfindung enthalten mindestens eine im pH-Bereich von 7 bis 9 wirksame Puffersubstanz, mindestens ein Tensid und gegebenenfalls Polyvinylpyrrolidon oder Polyvinylpolypyrrolidon und Salze, Komplexbildner, Proteinasen, Polyamine, β-Mercaptoethanol sowie weitere übliche Zusätze.

Überraschenderweise konnte gefunden werden, daß derartige erfindungsgemäße Extraktionspuffer in Verbindung mit geeigneten Adsorptionsmitteln erstmals die Isolierung von RNA in funktionaler Form aus quasi jedem beliebigen Pflanzenmaterial in einem deutlich vereinfachten Verfahren mit deutlich geringerem Zeitaufwand ermöglichen. Gegenüber den im Stand der Technik bekannten Isolierungsverfahren, die in der Regel mehr als 8 Stunden Aufarbeitungszeit erfordern, kann mit dem erfindungsgemäßen Kit und dem zugehörigen Verfahren innerhalb ca. einer Stunde hochreine, für molekularbiologische Arbeiten (bspw. RT-PCR) einsetzbare Ribonukleinsäure gewonnen werden. Zudem liegen die erzielbaren Ausbeuten überraschenderweise sehr hoch, so daß mit deutlich geringeren Mengen an Probenmaterial (ca. 30-100 mg), als bisher erforderlich war, gearbeitet werden kann. Das erfindungsgemäße Verfahren ermöglicht zudem die parallele Bearbeitung mehrerer Proben in gleichen, vorzugsweise geringen Volumina.

Von besonderem Interesse ist die Erfindung für Bäume, Sträucher und Zierpflanzen, also im Zusammenhang mit pflanzlichen Geweben, aus denen die Isolierung von funktionaler RNA auf einfache Weise und mit angemessenen Ausbeuten bislang praktisch nicht möglich war.

Als Puffersubstanz im Extraktionspuffer gemäß der vorliegenden Erfindung wird ein Tris(hydroxymethyl)aminomethansalz, insbesondere Tris-HCl bevorzugt. Weitere erfindungsgemäß verwendbare Puffer sind z.B. Acetate, Bicarbonate, bis-tris-Propan, Borate, Citrate, Dimethylmalonate, Glycinamide, Glycylglycine, Imidazole, Phosphate, Succinate und dergleichen.

Erfindungsgemäß bevorzugte Tenside umfassen kationische Tenside, insbesondere quartäre Ammoniumsalze vom Typ NR¹(R²)₃⁺, wobei R¹ ein Alkyl- oder Alkenylrest von C₈-C₁₈ und R² ein kurzkettiger Alkylrest ist, und besonders bevorzugt Cetyltrimethyl-ammoniumbromid (CTAB).

Der Gehalt an CTAB oder ähnlichen Tensiden im erfindungsgemäßen Extraktionspuffer liegt bei 0,1 bis 5 Gew.-%, bevorzugt bei 0,5 bis 4 Gew.-%, und besonders bevorzugt bei etwa 3 Gew.-%, bezogen auf die Gesamtzusammensetzung des Puffers.

Zur Entfernung phenolischer Substanzen enthält der Extraktionspuffer gemäß der vorliegenden Erfindung in einer bevorzugten Ausführungsform Polyvinylpyrrolidon (PVP) oder Polyvinylpolypyrrolidon (PVPP). Es wurde festgestellt, daß hohe PVP- bzw. PVPP-Konzentrationen die Nukleinsäureausbeute verringern, weshalb der PVP- bzw. PVPP-Anteil im Extraktionspuffer unter 10 % liegen sollte. Bevorzugt wird ein PVP- bzw. PVPP-Gehalt von 0,1 bis 5 Gew.-%, insbesondere von 0,5 bis 4 Gew.-%, und besonders bevorzugt von etwa 2 Gew.-%.

Dem erfindungsgemäßen Extraktionspuffer können zur Inaktivierung von Nucleasen Komplexbildner zugesetzt werden. Geeignet sind alle Komplexbildner, die in der Lage sind zweiwertige Metallkationen, wie sie in Enzymen üblicherweise vorkommen, zu binden. Bevorzugt wird EDTA und deren Salze.

Ebenso möglich und erwünscht ist der Zusatz von Polyaminverbindungen wie Putrescin, Spermin und Spermidin, wobei Spermidin bevorzugt wird.

Um Oxidation von SH-Gruppen in Proteinen zu verhindern, kann dem erfindungsgemäßen Extraktionspuffer ein Reduktionsmittel zugesetzt werden, vorzugsweise β-Mercapto-ethanol.

Die Anwendung des erfindungsgemäßen RNA-Isolierungs-Kits bzw. des Extraktions-puffers kann nach folgendem Vorgehensweise erfolgen:
Zur Isolierung von Ribonukleinsäuren aus Pflanzen- oder Pilzmaterial werden die folgenden Schritte angewandt: Nach der Zerstörung der Zellwände in üblicher Weise, z.B. Verreiben des Materials in flüssigem Stickstoff oder mit Trockeneis wird das Versuchsmaterial mit auf Inkubationstemperatur vorerwärmtem Extraktionspuffer versetzt, gut durchmischt und für ca. 5 Minuten bei erhöhter Temperatur zwischen 20° und 80 °C, vorzugsweise bei 65 °C, inkubiert. Anschließend werden ein geeignetes, nicht mit Wasser mischbares organisches Lösemittel oder Lösemittelgemisch sowie mindestens ein

Polysaccharide und andere Sekundärstoffe bindendes Adsorptionsmittel zugegeben. Geeignete Lösemittel sind z. B. Chloroform, Methylenchlorid, Dichlormethan, aliphatische lineare und cyclische Kohlenwasserstoffe, langkettige verzweigte und unverzweigte, gesättigte und ungesättigte Alkohole, und dergleichen. Bevorzugt werden Gemische aus Chloroform und Isoamylalkohol im Verhältnis 24:1. Das Lösemittelgemisch wird vorzugsweise auf etwa -20 °C gekühlt zugesetzt.

Bei stark gummihaltigen Pflanzen kann der zusätzliche Zusatz eines nichtionischen polymeren Adsorbens wie z.B. Amberlite® XAD-2 zur Entfernung von Latexanteilen nötig sein. Bevorzugt erfolgt der Zusatz gleichzeitig mit dem Adsorbens aus dem Kit. Pflanzen mit hohem Gehalt an phenolischen Zusätzen (z.B. Kiefer, Birke, Ahorn) können die zusätzliche Zugabe von Salzen, insbesondere CaCl₂ erforderlich machen. In ähnlicher Weise wird die Isolierung großer Mengen von RNA bei Anwesenheit schleimartiger Sekundärstoffe (wie sie bspw. in Lindenblüten und -blättern vorkommen) durch Zusatz von KCl und/oder Benzylchlorid begünstigt, wobei die Zugabe bevorzugt zusammen mit dem Adsorptionsmittel erfolgt.

Ebenso kann bei stark proteinhaltigen Pflanzen dem Extraktionspuffer Proteinase zugesetzt werden, vorzugsweise Proteinase K, um den Proteinabbau zu gewährleisten und so die Reinheit der isolierten Nukleinsäure zu erhöhen.

Anschließend wird die Mischung bei Raumtemperatur geschüttelt und dann zentrifugiert. Die nukleinsäurehaltige wäßrige Phase wird dekantiert und gegebenenfalls ein zweites Mal mit organischem Lösemittel extrahiert. Anschließend wird die Nukleinsäure aus der wäßrigen Phase in bekannter Weise, gegebenenfalls unter Verwendung von DNase und dazu notwendigen Enzympuffern, in reiner Form isoliert.

Der erfindungsgemäße Nukleinsäure-Isolierungs-Kit bzw. der erfindungsgemäße Extraktionspuffer können bei einer quasi unbegrenzten Vielzahl von Pflanzenspezies, Pflanzengeweben und Pilzen eingesetzt werden. Selbstverständlich eignen sich der erfindungsgemäße Kit bzw. Extraktionspuffer auch zur Isolierung von RNA aus weniger problematischen Materialien wie Bakterienzellen und tierischen Geweben.

Die nachfolgenden Beispiele zeigen einige Beispiele für die Anwendung der vorliegenden Erfindung und stellen keine Einschränkung auf bestimmte Anwendungsbereiche dar. Vielmehr kann man sich eine Vielzahl weiterer Anwendungsmöglichkeiten der vorliegenden Erfindung vorstellen.

### Beispiel 1: Herstellung eines Extraktionspuffers.

Für die Isolierung von RNA aus Pflanzen wird auf der Basis von reinem, nucleasefreien Wasser eine Mischung mit folgenden Mengen an Inhaltsstoffen zusammengestellt und autoklaviert:

| | |
|---|---|
| Tris-HCl (pH 8) | 40 mM |
| CTAB | 3 % |
| PVPP/PVP | 2 % |
| EDTA | 50 mM |
| NaCl | 2 M |
| Spermidin | 0,5 g/l |
| β-Mercaptoethanol | 2 % |

Die Zugabe des β-Mercaptoethanols folgt erst nach der Autoklavierung.

### Für die RNA-Isolierung aus Pflanzen bzw. Pilzen sind weiterhin notwendig:

Chloroform/Isoamylalkohol 24:1 (auf -20 °C gekühlt)
Nucleon PhytoPure Resin® der Firma Nucleon (Adsorptionsmittel)
Isopropanol (gekühlt auf 4 °C)
Ethanol, 70 % (auf -20 °C gekühlt)
DNase 1 (Pharmacia; 5U≈1,5 Kunitz Units)
DNase Puffer (10x): 400 mM Tris-HCl (pH 7,5), 60 mM MgCl₂
   gegebenenfalls (z.B. bei Pflanzenspezies mit hohem Phenol- oder Latexanteil):
20 % CaCl₂
1 M KCl
1 M Benyzlchlorid
XAD-2
Proteinase K 10 mg/ml

### Allgemeine Vorschrift für die Isolierung von RNA aus Pflanzen bzw. Pilzen:

Etwa 30-50 mg Pflanzen- (bzw. Pilz-) Material in einem 2 ml Eppendorfgefäß mit 500 µl auf 65°C erwärmtem Extraktionspuffer versetzen, gut mischen und 5 min bei 65 °C inkubieren. Dann 250 µl gekühltes Lösungsmittel bzw. -gemisch und 100 µl Adsorptionsmittel zugeben, gut mischen und 10 min bei Raumtemperatur schütteln lassen (bei ca. 1800 upm). Anschließend bei 4°C für 5 min bei 13000 upm zentrifugieren. Den wäßrigen Überstand abnehmen und je nach Bedarf, also optional, ein zweites Mal mit 250 µl Lösungsmittel bzw. -gemisch kurz mischen und anschließend für 2 min bei 4°C und 13000 upm zentrifugieren. Die gesammelten wäßrigen Überstände mit zwei Volumeneinheiten kaltem Isopropanol versetzen, mindestens 5 min auf Eis inkubieren und anschließend bei 4°C und 13000 upm für 5 min zentrifugieren. Das Pellet in 13 µl Wasser aufnehmen, 2 µl Enzympuffer und 5µl DNase 1 zugeben und 15 min bei 37 °C inkubieren.

Anschließend zwei Volumeneinheiten Isopropanol zugeben, mischen und bei 4°C und 13000 upm für 2 min zentrifugieren. Das Pellet mit 200 µl 70 %igem Ethanol versetzen. Nochmals zentrifugieren, das RNA-Pellet ca. 20 min trocknen lassen und schließlich in 50 µl Wasser oder Puffer (TE) aufnehmen.

### Beispiele 2-9: Isolierung von RNA aus Nutz- und Modellpflanzen:

Nach der oben angegebenen allgemeinen Vorschrift wurde mit Hilfe des Extraktions-puffers aus Beispiel 1 RNA aus folgenden Nutz- und Modellpflanzen isoliert:

| Beispiel | Pflanze | Besonderheiten | Zugabe |
|---|---|---|---|
| 2 | Gerste | | |
| 3 | Tomate | | |
| 4 | Kartoffel | | |
| 5 | Bohne (Blatt, Wurzel) | | |
| 6 | Bohne (Kotyledonen) | hoher Polysaccharidanteil, hoher Hintergrund | zusätzlich Proteinase K (100 µl 10 mg/ml) |
| 7 | Soja (Kotyledonen) | hoher Polysaccharidanteil, hoher Hintergrund | zusätzlich Proteinase K (100 µl 10 mg/ml) |
| 8 | Arabidopsis | | |
| 9 | Tabak | | |

### Beispiele 10-16: Isolierung von RNA aus Zierpflanzen:

Nach der oben angegebenen allgemeinen Vorschrift wurde mit Hilfe des Extraktions-puffers aus Beispiel 1 RNA aus folgenden Zierpflanzen isoliert:

| Beispiel | Pflanze | Besonderheiten | Zugabe |
|---|---|---|---|
| 10 | Tradescantia | | |
| 11 | Wasserpflanze (Aquarium) | | |
| 12 | Buntnessel | | |
| 13 | Christusdorn | | |
| 14 | Oleander | | |
| 15 | Benjaminfeige | hoher Latexanteil | XAD-2 |
| 16 | Stapelle | | |

### Beispiele 17-25: Isolierung von RNA aus Blüten:

Nach der oben angegebenen allgemeinen Vorschrift wurde mit Hilfe des Extraktions-puffers aus Beispiel 1 RNA aus folgenden Blüten isoliert:

| Beispiel | Pflanze |
|---|---|
| 17 | Usambaraveilschen |
| 18 | Gelber Hahnenfuß |
| 19 | Flieder |
| 20 | Apfel |
| 21 | Rose (Kardinal) |
| 22 | Pelargonie |
| 23 | Cyclamen |
| 24 | Gerbera |
| 25 | Petunie |

Keine Besonderheiten, Zugabe weiterer Stoffe nicht erforderlich.

### Beispiele 26-33 Isolierung von RNA aus Bäumen:

Nach der oben angegebenen allgemeinen Vorschrift wurde mit Hilfe des Extraktions-puffers aus Beispiel 1 die RNA aus folgenden Bäumen isoliert:

| Beispiel | Pflanze | Besonderheiten | Zugabe |
|---|---|---|---|
| 26 | Linde (Blatt, Blüte) | unbekannte Inhaltsstoffe | KCl (200 µl 1 M), Benzylchlorid (100 µl 1 M) |
| 27 | Hasel | | |
| 28 | Vogelbeere | | |
| 29 | Eiche | | |
| 30 | Birke | unbekannte Inhaltsstoffe | CaCl₂ (100 µl 20% w/v) |
| 31 | Ahorn | unbekannte Inhaltsstoffe | CaCl₂ (100 µl 20% w/v) |
| 32 | Buche | | |
| 33 | Kiefer (Nadeln, Phloem, Zapfen, Wurzel) | | besser mit CaCl₂-Zugabe |

### Beispiele 34-35: Isolierung von RNA aus Pilzen:

Nach der oben angegebenen allgemeinen Vorschrift wurde mit Hilfe des Extraktions-puffers aus Beispiel 1 die RNA aus folgenden Pilzen isoliert:

| Beispiel | Pflanze |
|---|---|
| 34 | Phanerochaete chrysosporium |
| 35 | Lophodermium picea |

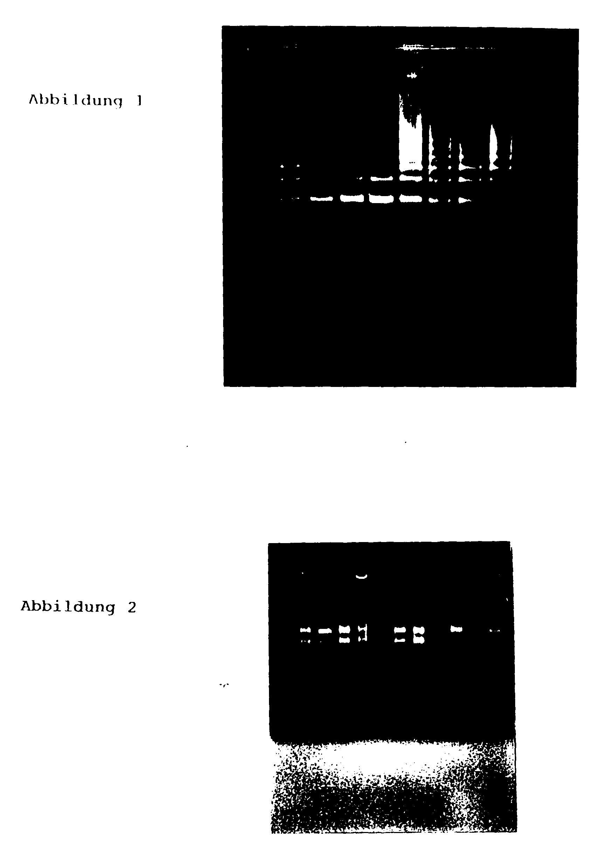

## Patentansprüche

1. RNA-Isolierungs-Kit umfassend einen Extraktionspuffer und wenigstens ein Adsorptionsmittel,
**dadurch gekennzeichnet**, daß der Extraktionspuffer mindestens eine im pH-Bereich von 7-9 wirksame Puffersubstanz, mindestens ein Tensid und gegebenenfalls Polyvinyl-pyrrolidon (PVP) oder Polyvinylpolypyrrolidon (PVPP) und weitere übliche Inhaltsstoffe wie Salze, Komplexbildner, Proteinasen, Mercaptoethanol, Polyamine und dergleichen enthält und das Adsorptionsmittel in der Lage ist, Polysaccharide zu binden.

2. RNA-Isolierungs-Kit gemäß Anspruch 1,
**dadurch gekennzeichnet**, daß das Adsorptionsmittel veresterbare funktionelle Gruppen umfaßt.

3. RNA-Isolierungs-Kit gemäß Anspruch 1 oder 2,
**dadurch gekennzeichnet**, daß das Adsorptionsmittel freie Borsäuregruppen umfaßt.

4. RNA-Isolierungs-Kit gemäß einem der vorangehenden Ansprüche,
**dadurch gekennzeichnet**, daß das Adsorptionsmittel eine Siliciumdioxidmatrix umfaßt.

5. RNA-Isolierungs-Kit gemäß einem der vorangehenden Ansprüche,
**dadurch gekennzeichnet**, daß die Puffersubstanz ein vom Tris(hydroxymethyl)aminomethan abgeleitetes Salz, insbesondere Tris-HCl, umfaßt.

6. RNA-Isolierungs-Kit gemäß einem der vorangehenden Ansprüche,
**dadurch gekennzeichnet**, daß das Tensid ein kationisches Tensid, vorzugsweise ein quartäres Ammoniumsalz, und besonders bevorzugt CTAB ist.

7. RNA-Isolierungs-Kit gemäß einem der vorangehenden Ansprüche,
**dadurch gekennzeichnet**, daß der Tensidgehalt im Extraktionspuffer bezogen auf die Gesamtzusammensetzung bei 0,1 bis 5 Gew.-%, bevorzugt bei 0,5 bis 4 Gew.-%, und besonders bevorzugt bei etwa 3 Gew.-% liegt.

8. RNA-Isolierungs-Kit gemäß einem der vorangehenden Ansprüche,
**dadurch gekennzeichnet**, daß der Gehalt an PVP oder PVPP im Extraktionspuffer bezogen auf die Gesamtzusammensetzung bei 0,1 bis 5 Gew.-%, bevorzugt bei 0,5 bis 4 (Gew.-%, und besonders bevorzugt bei etwa 2 Gew.-% liegt.

9. RNA-Isolierungs-Kit gemäß einem der vorangehenden Ansprüche,
**dadurch gekennzeichnet**, daß der Extraktionspuffer EDTA und/oder Spermidin enthält.

10. RNA-Isolierungs-Kit gemäß einem der vorangehenden Ansprüche,
**dadurch gekennzeichnet**, daß der Extraktionspuffer β-Mercaptoethanol enthält.

11. Extraktionspuffer zur Isolierung von RNA, umfassend mindestens eine im pH-Bereich von 7-9 wirksame Puffersubstanz, mindestens ein Tensid und gegebenenfalls Polyvinylpyrrolidon (PVP) oder Polyvinylpolypyrrolidon (PVPP) und weitere übliche Inhaltsstoffe wie Salze, Komplexbildner, Proteinasen, Mercaptoethanol, Polyamine, und dergleichen.

12. Extraktionspuffer gemäß Anspruch 11,
**dadurch gekennzeichnet**, daß die Puffersubstanz ein vom Tris(hydroxymethyl)aminomethan abgeleitetes Salz, insbesondere Tris-HCl, umfaßt.

13. Extraktionspuffer gemäß Anspruch 11 oder 12,
**dadurch gekennzeichnet**, daß das Tensid ein kationisches Tensid, vorzugsweise ein quartäres Ammoniumsalz, und besonders bevorzugt CTAB ist.

14. Extraktionspuffer gemäß einem der Ansprüche 11 bis 13,
**dadurch gekennzeichnet**, daß der Tensidgehalt im Extraktionspuffer bezogen auf die Gesamtzusammensetzung bei 0,1 bis 5 Gew.-%, bevorzugt bei 0,5 bis 4 Gew.-%, und besonders bevorzugt bei etwa 3 Gew.-% liegt.

15. Extraktionspuffer gemäß einem der Ansprüche 11 bis 14,
**dadurch gekennzeichnet**, daß der Gehalt an PVP oder PVPP im Extraktionspuffer bezogen auf die Gesamtzusammensetzung bei 0,1 bis 5 Gew.-%, bevorzugt bei 0,5 bis 4 Gew.-%, und besonders bevorzugt bei etwa 2 Gew.-% liegt.

16. Extraktionspuffer gemäß einem der Ansprüche 11 bis 15,
**dadurch gekennzeichnet**, daß der Extraktionspuffer EDTA und/oder Spermidin enthält.

17. Extraktionspuffer gemäß einem der Ansprüche 11 bis 16,
**dadurch gekennzeichnet**, daß der Extraktionspuffer β-Mercaptoethanol enthält

18. Extraktionspuffer gemäß einem der Ansprüche 11 bis 17,
**gekennzeichnet durch** einen Gehalt von 40 mM Tris-HCl, 3 % CTAB, 2 % PVPP, 50 mM EDTA, 2 M NaCl, 0,5 g/l Spermidin und 2 % β-Mercaptoethanol.

19. Verfahren zur Isolierung von Nukleinsäuren aus biologischem Material, umfassend die Schritte:
a) Mischen des biologischen Materials mit einem Extraktionspuffer gemäß einem der Ansprüche 11 bis 18,
b) Inkubieren der Mischung bei erhöhter Temperatur,
c) Zugabe von Lösungsmittel bzw. -gemisch und mindestens einem Adsorptionsmittel aus dem RNA-Isolierungs-Kit gemäß einem der Ansprüche 1 bis 10,
d) Abtrennen der wäßrigen Phase und Isolierung der darin enthaltenen gereinigten Nukleinsäuren nach üblichen Methoden.

20. Verfahren nach Anspruch 19,
**dadurch gekennzeichnet**, daß das Lösungsmittel bzw. -gemisch ein Gemisch von Chloroform und Isoamylalkohol, vorzugsweise im Verhältnis 24:1, ist.

21. Verfahren nach Anspruch 18 oder 19,
**dadurch gekennzeichnet**, daß es sich dem biologischen Material um pflanzliches Material handelt.

22. Verwendung des Extraktionspuffers gemäß einem der Ansprüche 11 bis 18 zur Isolierung von RNA aus biologischem Material, insbesondere aus Pflanzenmaterial.

23. Verwendung eines Adsorptionsmittels gemäß einem der Ansprüche 1 bis 4 zur Isolierung von RNA aus biologischem Material, insbesondere aus Pflanzenmaterial.
